# EUROPEAN PATENT SPECIFICATION

(11) **EP 1 656 120 B1**
(45) Date of publication and mention of the grant of the patent: **28.11.2012**
(21) Application number: 04743442.8
(22) Date of filing: 19.07.2004
(51) Int. Cl.: A61K 9/50, A61K 9/70, A61K 33/14, A61K 31/60, A61K 31/155

(54) **COMPOSITIONS COMPRISING COMPONENTS COATED WITH A LIQUID IMPERMEABLE BUT GAS PERMEABLE LAYER, USE THEREOF FOR TREATING CUTANEOUS AND OTHER EXOCRINE GLAND DISEASES**
ZUSAMMENSETZUNGEN MIT KOMPONENTEN, DIE MIT EINER FLÜSSIGKEITSUNDURCHLÄSSIGEN, ABER GASDURCHLÄSSIGEN SCHICHT ÜBERZOGEN SIND, IHRE VERWENDUNG ZUR BEHANDLUNG VON HAUTERKRANKUNGEN UND ANDEREN ERKRANKUNGEN EXOKRINER DRÜSEN
COMPOSITIONS COMPRENANT DES COMPOSANTS ENROBES D'UNE COUCHE PERMEABLE AUX GAZ MAIS IMPERMEABLES AUX LIQUIDES, LEUR UTILISATION POUR LE TRAITEMENT D'AFFECTIONS DE LA PEAU ET DE LA GLANDE EXOCRINE

(30) Priority: 19.07.2003 GB 0316940; 20.11.2003 GB 0327006
(43) Date of publication of application: 17.05.2006
(73) Proprietor: Ward, Warren, Gwynedd LL19 8PG (GB)
(72) Inventor: Ward, Warren, Gwynedd LL19 8PG (GB)
(74) Representative: Robertson, James Alexander
(86) International application number: PCT/GB2004/003104
(87) International publication number: WO 2005/013942

(56) References cited:
- WO-A-03/005995
- GB-A- 2 119 244
- US-A- 4 970 081
- US-A1- 2001 042 932
- SAITO K ET AL: "EFFECT OF IONIC INTERACTION ON THE ENTRAPPING OF DRUG INTO POROUS MICROSPHERES AND DRUG RELEASE CHARACTERISTICS" CHEMICAL AND PHARMACEUTICAL BULLETIN, PHARMACEUTICAL SOCIETY OF JAPAN. TOKYO, JP, vol. 35, no. 5, May 1987 (1987-05), pages 2045-2051, XP001182583 ISSN: 0009-2363

## Description

The present invention relates to the treatment of medical conditions and particularly medical conditions at least partially characterised by blockage or other malfunction of exocrine glands, particularly ducts of exocrine glands, and especially ducts of sweat glands.

People in advanced western societies such as the UK and USA are increasingly likely to suffer from a number of chronic diseases (e.g. essential hypertension, asthma, inflammation of the gastro-intestinal tract) and despite expensive medical intervention the incidence of such diseases continue to rise.

Several of these chronic conditions are known to be related to the blockage of ducts of exocrine glands. For example, it is well known that the blockage of sweat gland ducts and retained sweat can lead to Miliaria, an acute inflammatory skin condition better known as Prickly Heat.

Adult humans are estimated to have between 2 and 4 million sweat glands, with ducts to the skin surface. Sweat is known to be a fluid consisting mainly of water, with waste products such as urea, plus sodium and other salts.

Miliaria occurs when sweat gland ducts are obstructed. As a consequence, sweat does not reach the skin surface and is trapped in the epidermis or dermis, where it causes a prickling sensation often accompanied by severe itching. Even when ducts are blocked, the sweat glands continue to output fluid and just below the position of the blockage, the pressure of the sweat ruptures the duct and forces sweat into the surrounding skin. If sweat increases as a result of emotion, heat or exercise, then the amount of damage to the surrounding skin may be even greater.

Depending on the depth in which the obstruction occurs, different types of lesions appear. Ductal obstruction in the uppermost epidermis results in Miliaria crystallina with asymptomatic superficial vesicles, whilst obstruction with inflammation occurring deeper in the epidermis leads to Miliaria rubra, which is characterised by red lesions and appears as pruritic and tender red macules or papules. This type of Miliaria can become infected and pustular and is very unpleasant. Current treatment consists of remaining in a cool environment for some weeks, and the topical application of pure lanolin, which has a temporary effect.

If duct blockage occurs in the upper dermis, in a layer richly provided with nerve endings (the itch layer), then there is painful and pruritic inflammation. This is a previously unknown Miliaria type identified by the inventor (designated Miliaria type 3), and which is believed to leads to atopic dermatitis or eczema.

In the deepest and most severe form of Miliaria, called Miliaria profunda, ductal obstruction occurs near the entrance of the duct into the dermal papillae resulting in subtle asymptomatic, flesh coloured papules. In Miliaria profunda the sweat spreads into the surrounding skin and, unseen and unnoticed, ruptures adjacent blood capillaries. The inventor believes this to be a potential cause of essential hypertension, as more and more ruptured capillaries cause pressure in the blood circulation to rise, generally continuing to rise with age. The rupture of the capillaries may sometimes trigger the formation of dangerous thrombi, particularly in the lower legs.

Persons who are unacclimatised to heat but who remain in high ambient temperatures, or who exert themselves in high temperatures, are likely to suffer extensive acute Miliaria profunda. The blockage of many gland ducts disables the ability of the body to cool properly by sweat evaporation, potentially leading to heat exhaustion or heat stroke.

Miliaria can also occur where exercise is undertaken or there is heat exposure in persons wearing occlusive clothing. This is a common cause of various types of Miliaria for example in occupations such as mining, fire fighting, catering and other physical jobs in hot conditions.

Human facial skin is thin, being only about 10% of the thickness of skin on the back. Blockages in the sweat ducts of facial skin may result in any type of skin Miliaria, and the inventor found that a common type is a combination of Miliaria rubra and Miliaria type 3 (see above). This combination Miliaria is often temporary but may eventually cause chronic erythema, flushing, and blushing with cyclical crops of pustules and papules. Ultimately, particularly in men, this can lead to distinctive tissue hyperplasia and disfiguring phyma, such as rhinophyma, a bulbous hypertrophy of the nose. The inventor believes this combination of Miliaria types may also result in rosacea featuring red lines, or telangiectasia, said to be characteristic thereof. Blockages of the sebaceous exocrine ducts of facial and other skin causes the common condition of acne vulgaris which often accompanies roseacea.

Furthermore, the inventor has found that, particularly in females, blockage of exocrine ducts may cause sweat filled vacuoles in the dermis. If the affected area of skin is subjected to pressure, for example the sitting contact area of the thighs and the buttocks, adipose tissue is extruded through the connective tissue of the border into the dermis to produce a characteristic irregular dimpled effect, better known as cellulite.

The inventor has found that the disease of psoriasis may be a type of Miliaria where as a result of trauma to the skin a microbial infection becomes trapped beneath sweat duct blockages.

The inventor has also recognised that many other diseases are affected by the blockage or other malfunction of exocrine ducts in tissues other than the skin.

The inventor believes that the blockage of exocrine ducts on the head may cause constriction or interruption of blood circulation to the brain which itself may result in migraine. Furthermore, such chronic blockage may possibly lead to various types of neurodegeneration.

The inventor has established that blockage of exocrine glands in the mucosal surfaces of the lungs contributes to asthma. The inventor also believes that allergic rhinitis or hay fever is caused by a blockage of exocrine glands of the mucosal surface of the interior of the nose.

Any blockage of exocrine glands may affect the skin of the breasts, which are well supplied with sweat glands and ducts. Also, female breasts have some degree of fluid secretion activity from milk glands throughout adult life, even when apparently not lactating. Additional milky discharge can also be due to drugs or hormones which stimulate milk production, or to mechanical stimulation of the nipple. The inventor believes that milk gland ducts are subject to blockage and rupture in the same manner as eccrine sweat ducts and that such blockages cause lumps, cysts, pain and tenderness in the female breast. Accordingly, the inventor believes these conditions are influenced by exocrine function.

Similarly, the inventor believes that blockage of the ducts of the prostate gland in men causes enlargement and pressure.

Furthermore, dry eye conjunctivitis and glaucoma can be caused by the blockage of exocrine ducts; and blockage or malfunction of exocrine glands of the mucosal surfaces of the gastro-intestinal tract is a cause of many inflammatory diseases including gastritis and colitis. Moreover, the inventor has found that such inflammation of the gastro-intestinal tract, particularly inflammation of the intestines, might induce autoimmune responses which may cause diseases such as Crohn's disease, rheumatoid arthritis, osteoarthritis, systemic lupus erythematosus, ankylosing spondylitis, multiple sclerosis, motor neurone disease, polycystic ovarian syndrome, mitral valve syndrome, diabetes type 1, scleroderma, autoimmune thyroiditis, Graves disease and many others.

The blockage or malfunction of exocrine ducts of the lungs and gastro-intestinal tract is believed to involve a loss of mucosal surface innate immunity, thereby leaving the subject open to air or fluid borne infection. The inventor therefore recommends that such conditions should be cleared up in persons likely to be exposed to pathogens, such as patients and staff in hospitals and before any medical procedures or medication programmes, including vaccinations, are commenced.

Also, especially in elderly persons who have extensive blockage of exocrine glands within skin, a lack of protection by skin anti-microbial peptide may lead to the easy entry of resident or common pathogens through the skin, potentially leading to severe infections.

It is well known that the disease of type 2 diabetes is associated with hypertension and with serum hyperglycaemia which is otherwise normally controlled by insulin hormone. It is the contention of the inventor that insulin producing pancreatic ducts function in the same manner as exocrine ducts. The excess output of insulin stimulated by hyperglycaemia is modified in passage through ducts and the modified insulin is no longer effective. This is "insulin resistance", known to be a cause of type 2 diabetes.

Furthermore the inventor believes that excess output of the hormones of the stomach, intestines, liver and pancreas results in a partial loss of normal function of these hormones, leading to a loss of control of appetite. Thus any weight loss programme for obesity can be assisted by including treatment of hormone producing exocrine ducts.

It is the obj ect of the present invention to provide treatment for Miliaria and other disorders partially caused by the blockage or other malfunction of exocrine ducts.

According to the present invention, there is provided a composition for use as defined in independent claim 1, a patch as defined in claim 16, a device as defined in claim 21, a preparation as defined in claim 26, the use as defined in clam 27 and a method of manufacture as defined in claim 28. Further preferable features are defined in the appended dependent claims.

According to a first aspect of the present invention there is provided the use of sodium chloride formulated such that it cannot cross epithelial barriers in the manufacture of a medicament for the treatment of medical conditions at least partially characterised by blockage or other malfunction of exocrine glands. For example, the blocking or malfunctioning may be of an exocrine duct.

In particular, the sodium chloride may be coated with an agent that forms a liquid impermeable but gas permeable layer. In this and other aspects of the present invention, the coated agent may prevent passage through the coating of the substance or agent (e.g. sodium chloride) encapsulated within it. The coating may be considered to be an encapsulation of the sodium chloride (or other substances detailed below which are coated). Thus administration of the substance (the sodium chloride or other agents as detailed below, coated with / encapsulated by the agent) to a patient may result in no metabolic change to, chemical change to, or diminution of, the sodium chloride (or other substance as detailed below).

The coating surrounding the sodium chloride (e.g. sodium chloride crystals or granules) may be any liquid impermeable but gas permeable barrier which prevents the sodium chloride from passing into (e.g. when ingested) or onto the body. The agent may be ceramic (e.g. a clay or an inorganic non-metallic material), a polymer or a natural wax.

In the case of sodium chloride, it can be encapsulated to form a sphere, for example of a diameter of 1-10 mm. For example, the sphere may comprise sodium chloride crystals coated with bees wax hardened with cornstarch and talc.

Sodium chloride or common salt (chemical formula NaCl) occurs naturally in many parts of the world as the mineral, halite and as mixed evaporates in salt lakes, by mass, sodium chloride is 60.663% elemental chlorine (Cl) and 39.337% sodium (Na).

The sodium chloride may be in crystal form. Sodium chloride crystals are cubic in form and represent a preferred form of sodium chloride for use according to the first aspect of the invention.

It is more preferred that the sodium chloride is encapsulated by a coating agent. Such an agent should encapsulate sodium chloride crystals such that the coating is liquid impermeable but gas permeable. The inventor has termed such encapsulated crystals "Sensezero Therapeutic Inert Agent".

Encapsulated sodium chloride crystals represent an important feature-of the present invention. Therefore, according to a second aspect of the present invention there is provided medically efficacious compound coated with an agent that forms a liquid impermeable but gas permeable layer for use as a medicament.

It will be appreciated that the coating of the second aspect of the invention may be applied to a number of medically useful compounds. It is most preferred that the compound is sodium chloride.

Prior art reveals the following compositions related to the general idea of present application.

GB2119244 refers to a dosage formulation (tablet, capsule) comprising potassium chloride and salicylic acid or a salicylate salt surrounded by a polymeric coating (vinylchloride-vinylacetate copolymer and magnesium lauryl sulphate). The osmotic devices comprise a semipermeable wall of cellulose acetate.

Saito et al. (1987) report polymer-coated porous microspheres containing salicylic acid as a model drug.

WO03005995 discloses a controlled release formulation comprising a) a core of metformin b) a layer of pioglitazone hydrochloride on at least a portion of a surface of said core c) combining a modulating polymer material with at least one of said metformin or said pioglitazone hydrochloride. The composition is suitable for the treatment of diabetes as exocrine gland disease.

In US4970081 coated aspirin granules which are compressed to tablets are shown, whilst the coating comprises a copolymer of ethyl acrylate and methyl methacrylate, hydroxypropylmethyl cellulose.

US2001042932 describes microcapsules on the basis of salicylic acid encapsulated in PVP. Hydrophobic protein microspheres are coated with bioadhesive polymers by phase inversion (poly (fumaric-co-sebacic acid)).

According to the various aspects of the present invention, the coating may be any liquid impermeable but gas permeable barrier which prevents the sodium chloride (or other substances detailed below) from passing into (e.g. when ingested) or onto the body. The agent may be ceramic (e.g. a clay or an inorganic non-metallic material), a polymer or a natural wax. Thus sodium chloride crystals or granules can be coated with beeswax hardened with cornstarch and talc.

It should be noted that although sodium chloride is not normally considered to be a medically efficacious substance, when used in the various aspects of the present invention, it is medically efficacious.

In a preferred embodiment of the second aspect of the present invention, sodium chloride is encapsulated in a sphere with an approximate diameter of 1 mm to 10 mm, preferably 3 mm to 8 mm, but mostly preferred 6 mm. The sphere may comprise sodium chloride crystals (which may be compressed) coated with beeswax hardened with small amounts of cornstarch and talc.

The coating of beeswax hardened with small amounts of cornstarch and talc is distinct from prior art tablet formulations and suchlike which use wax to coat or encapsulate a medicament (for example wax polishing of tablets). In such cases, the wax is water permeable and is used to effect a slow or delayed release of the medicament, or to enhance the appearance of the medicament. This is in contrast to the present invention in which the coating (even in the case of a wax-based coating) is water impermeable but gas permeable.

The hardening of the beeswax with cornstarch and talc effects this required water impermeability.

It should be noted that normal dietary sodium chloride is not effective according to the present invention because such salt is able to be absorbed into the body by gastro-intestinal tract epithelia upon ingestion. Salt used according to the present invention is prepared such that this may not occur (e.g. according to the second aspect of the invention).

The following medical conditions are amongst the disorders that may be treated (prophylactically or when symptoms arise) by the use of sodium chloride according to the present invention:
Miliaria crystallina; Miliaria rubra; Miliaria type 3; Miliaria profunda; Chronic erythema; Flushing and blushing with cyclical crops of pustules and papules; Tissue hyperplasia; Disfiguring phyma; Rhinophyma; Rosacea; Telangiectasia; Essential hypertension; Migraine; Neurodegeneration; Cellulite; Asthma; Allergic rhinitis; Hay fever; Atopic eczema; Lumps and cysts of the breasts; Prostate gland enlargement; Dry eye conjunctivitis; Glaucoma; Inflammation of the gastro-intestinal tract; Gastritis; Colitis; Crohn's disease; Rheumatoid arthritis; Osteoarthritis; Systemic lupus erythematosus; Ankylosing spondylitis; Multiple sclerosis; Motor neurone disease; Polycystic ovarian syndrome; Mitral valve syndrome; Diabetes type 1; Scleroderma; Autoimmune thyroiditis; Graves disease; Diabetes type 2; Hypertension associated with diabetes type 2; Acne vulgaris; and Obesity.

Herein, by "treatment" is meant any treatment which is designed to cure, alleviate, remove or lessen the symptoms of, or prevent or reduce the possibility of contracting any disorder or malfunction of the human or animal body. The term "treatment" also includes the treatment of e.g. skin conditions such as cellulite (which may be considered to be cosmetic and not a bodily disorder or malfunction) and the maintenance or promoting of optimum health or cosmetic appearance of the human or animal body. Similarly, reference herein to "medically efficacious" substances is to substances which can be used to effect a treatment. Therefore a medically efficacious substance includes substances which can be used to effect the therapy or prophylaxis of a condition.

A preferred method for general treatment of the abovementioned conditions involves the application of sodium chloride so that it is in proximity to the blocked exocrine gland duct. It will be appreciated that the precise way in which sodium chloride is formulated and administered will depend on the individual conditions to be treated.

It will also be appreciated that the amount of sodium chloride that is required is determined by biological activity and bioavailability which in turn depends on the mode of administration, the physicochemical properties of any agent employed to coat the salt and whether sodium chloride is being used as a monotherapy or in a combined therapy. The frequency of administration will also be influenced by a number of factors and particularly the health status of the subject being treated.

Optimal dosages to be administered may be determined by those skilled in the art, and will vary with the particular condition being treated, the strength of the preparation, the mode of administration, and the advancement of the disease condition. Additional factors depending on the particular subject being treated will result in a need to adjust dosages, including subject age, weight, gender, diet, and time of administration.

According to the present invention, the treatment of different types of Miliaria or other disorders described above may involve placing sodium chloride crystals in proximity to skin.

This may be achieved by any method of adhering salt crystals to skin but preferably avoiding direct contact between the salt and the skin. Preferred methods include the use of adhesive tape and Velcro bandages. However, the most preferred way of placing sodium chloride in proximity of the skin is by formulation of a patch. The patch may be a patch of any type of sticking device provided it holds sodium chloride crystals close to the skin. The inventor has termed such patches "Sensezero Therapeutic Inert Patches".

Such patches represent an important feature of the present invention and therefore in a third aspect of the present invention there is provided a patch suitable for adherence to skin containing sodium chloride adapted for use in the treatment of medical conditions at least partially characterised by the blockage or other malfunction of exocrine glands.

In a preferred embodiment of the third aspect of the present invention, the patch may be any type of sticking plaster suitable for adherence to skin, more preferably a water resistant plaster and most preferably a hypoallergenic water resistant plaster. The patch may be of any shape, however preferably it is in the form of a figure eight.

According to a most preferred embodiment of the third aspect of the present invention there are provided two spherical granules of pure sodium chloride on the patch and preferably, the sodium granules are held at a distance of 1 mm to 1000 mm apart, but most preferably the granules are held at 30 mm apart. Preferably, the granules may be of 1-10 mm diameter but more preferably granules are of 2.5 mm diameter and have a coating. This coating is preferably as described by the second aspect of the present invention and is composed of water impermeable but gas permeable material which prevents delivery of the sodium chloride across the skin.

Another preferred method of placing sodium chloride crystals in proximity to skin is the use of a device which temporarily and at intervals places sodium chloride crystals against skin for a therapeutic purpose. The inventor has termed this "Sensezero Therapeutic Device".

Such a device represents an important feature of the present invention and therefore in a fourth aspect of the present invention there is provided a device consisting of a holder adapted for holding a medically efficacious compound, an energy source and an actuator driven by the energy source for temporarily and at intervals placing the compound against the skin of a subject. The device may be for treatment of conditions at least partially caused by the blockage or other malfunction of exocrine glands.

According to the fourth aspect of the present invention, such a device may be a miniature encased device made from any material, preferably made from plastic. The device may be worn anywhere on the body but more preferably on the abdomen or thorax of a subject. Within the casing there may be source of energy such as a low voltage battery in addition to an electronic timer. The actuator may be a spring return push-rod solenoid. The device may be adapted to hold sodium chloride. Preferably, pure sodium chloride crystals are placed in the holder. The sodium chloride may be coated with a coating according to the second aspect of the present invention. Such coated sodium chloride is water impermeable but gas permeable and thereby prevents delivery of salt across the skin.

Daily doses may be given as a single administration. Alternatively, the sodium chloride used may require administration twice or many times during a day. As an example, sodium chloride according to the invention may be administered by wearing the device continuously for as many days as required the device being activated for a few minutes in each hour. Alternatively a patient may take a daily dose comprising one patch which is replaced in a different position on the skin after each 24 hours.

Conditions at least partially characterised by a blockage or other malfunction of exocrine ducts may be treated with any one of the approaches encompassed by the first, second, third and fourth aspects of the present invention, For instance, a patch according to the third aspect of the invention may be applied in the abdominal region.

According to the present invention, the treatment of different types of Miliaria or other disorders at least partly characterised by blockage or other malfunction of exocrine ducts may involve placing sodium chloride crystals in proximity to skin. All conditions (skin and non-skin) may be treated in the same way (e.g. with a patch according to the third aspect of the invention on the skin, or alternatively use of the device according to the fourth aspect of the invention). Treatment can be reinforced and enhanced by simultaneously using a coated compound according to the second aspect of the invention which can also be used as a monotherapy.

Skin disease on the face, legs or arms may be treated by placing a patch according to the third aspect of the invention or device according to the fourth aspect of the invention on the abdomen or thorax, on unaffected skin if possible. This is preferably done with the additional use of a coated compound according to the second aspect of the invention in most cases. Non-skin disease may be treated in the same way. Some therapists might want to use a patch according to the third aspect of the invention or a device according to the fourth aspect of the invention or a coated compound according to the second aspect of the invention as a monotherapy. Following successful treatment of a disease, one of the three might be used periodically as a prophylactic.

However, it is preferred that the treatment of any of the conditions is enhanced synergistically by introducing into the body, orally or otherwise, sodium chloride in the form defined by the second aspect of the invention.

It will be appreciated that the invention (in all its aspects) is particularly useful for treating human subjects. However the subject may be any other mammal of veterinary interest.

Although the inventor does not wish to be bound by any hypothesis, it is believed that sodium chloride can be used for the treatment of medical conditions at least partially characterised by blockage or other malfunction of exocrine ducts for the following reasons.

The inventor has considered the behaviour of modern humans in advanced societies and has found that a change from an original genetic habituation (i.e. the status of Man in a natural habitat) to an adverse habituation (i.e. Man's unnatural or modem habitat) can result in several health problems including Miliaria disease and those mentioned above. It is believed that such change may manifest in an altered physiology of exocrine glands and particularly sweat glands.

The inventor believes that normal sweat glands output sweat either at a basal level or at a higher level. This dual status of sweat glands is believed to be the natural state and the inventor has named this status as original genetic habituation of sweat glands.

In this natural state all sweat glands continuously produce sweat at the basal level. In general, increasing exercise or exposure to heat causes more and more sweat glands to become involved at the higher level. Regular vigorous exercise or exposure to heat is essential for maintaining the genetic habituation status of sweat ducts, as they lead to abundant flow of sweat resulting in clear ducts due to the physical force of fast flowing fluid.

Persons who do not exercise regularly, who encounter emotions like stress and who live in artificial and temperate climates experience repeated episodes of slightly greater sweat output than the basal level. The inventor believes that, as a consequence, reabsorption of sodium by the duct becomes fixed at a slightly greater level causing the duct to remain in a state of adverse habituation even though the stimulus to sweat ceases. This acquired state predispositions an affected person to disorders related to the blockage of sweat glands since the salinity of the sweat sinks below the levels required for efficient skin protection against microbial pathogens by anti-microbial peptide. This enables microbes to enter ducts, which then become blocked by an ensuing immune reaction.

During the initial stages of investigation of potential treatments of the abovementioned medical conditions, the inventor considered oceanic unicellular prokaryotes which existed at the beginning of the evolutionary time. These, like modem cells, actively transport sodium in and out to maintain a lower sodium internal environment. Since any body of water containing dissolved salts is subject to changes of concentration following natural flows and stratification, the inventor concluded that, for survival, the earliest prokaryotes must have been able to detect internal and external elements of dissolved molecules, and act on the information. The inventor termed this detection ability "sensezero".

The inventor then pictured the human body enveloped in fluid, with the skin and the lungs in contact with fluid in the form of a gas, i.e. air, and the gastro intestinal tract in contact with liquid. The inventor concluded that, if the body senses the presence of surplus sodium in both the gas and the liquid environment, exocrine glands in adverse habituation are free to reset to genetic habituation.

Thus, according to the present invention, the basis for the treatment and prophylaxis of Miliaria and diseases mentioned above is to provide in the air and in the liquid environment of the body an amount of sodium, which appears to indicate a surplus to allow resetting to genetic habituation. Furthermore, to prevent desensitisation of the body to sodium chloride and the wearing off of the therapeutic effect, the inventor found that treatment and prophylaxis have to be arranged so that the body can sense new additional sodium salt without absorbing it. Accordingly, salt used according to the present invention should be made not to cross epithelial barriers.

Also it is now known that in multi-cellular animals complex cell signalling systems are used for motility, for survival and apoptosis, for example, and that these signals affect or control the maintenance of health and the progress of disease.

The inventor has also noted that many drugs which are formulated to pass into body circulation are not in fact metabolised in the body, but appear to achieve their therapeutic effect by their presence, and are then excreted unchanged.

The inventor therefore concluded that many drugs could have a therapeutic effect by being present in the environment of the body, but without entering circulation. The environment of the body he considered to be fluid. In the case of the skin and lungs the fluid is a gas i.e. air, and in the case of the gastrointestinal tract it is liquid.

The inventor does not wish to be bound by the hypothesis, but believes that the presence of a drug in the body environment but not in body circulation influences cell signalling and sensezero, and can therefore have a desired therapeutic effect without the drug being in circulation.

Thus as well as being applicable to sodium chloride, the above findings and each of the above aspects of the invention are also applicable to medicaments, patches and devices incorporating substances other than sodium chloride. In particular, the sodium chloride in the above described aspects of the present invention may be replaced by capsaicin, metformin, salicylic acid, or a derivative of salicylic acid thereof. Additionally, the sodium chloride may be replaced by a substance endogenous to the body (e.g. the human or animal body as appropriate, depending upon the intended recipient of any medication or treatment), a food substance, or a drug.

Many thousands of compounds are currently manufactured for use for therapeutic purposes. Naturally occurring substances, for example plant material, are also prepared for use for therapeutic purposes. For the present purpose, these compounds and substances are termed "drugs".

Drugs are almost always compounds foreign to the body. As such, they, unlike endogenous substances, are not continually being formed and eliminated. Drug absorption, bioavailability, distribution, and elimination are therefore determinants of onset, duration, and intensity of drug effect.

Drug absorption in mammals is determined by their physicochemical properties, their formulations and routes of administration. The actual dosage forms (e.g. tablets, capsules, solutions) consisting of the drug and suitable excipients are formulated to be administered by various routes including oral, buccal, sublingual, rectal, parenteral, dermal and inhalational. A prerequisite to absorption is drug dissolution. For example, solid drug products like tablets disintegrate and disaggregate quickly or slowly, but absorption can only occur after drugs enter solution.

Drugs are designed to enter systemic circulation to have the desired effect. Thus those skilled in the art of drug preparation are concerned to achieve effective transport across biological barriers, to control transit times, dissolution and absorption, and to maximise bioavailability in circulation and at the place of the therapeutic target. Some drugs cross the cell membrane to enter the cell itself.

Drugs in circulation may also metabolise in the body in a wide range of chemical reactions including oxidation, reduction, hydrolysis, hydration, conjugation, condensation and isomerisation and these reactions have to be carefully predicted. Unwanted metabolites may be difficult to eliminate from the body. There may also be harmful interactions with other drugs or with endogenous substances.

Drugs in circulation can accumulate over time in tissues or body compartments and thus cause undesirable effects. Drugs in circulation may penetrate areas where they would be harmful, such as across the blood-brain barrier or the placenta.

Therefore great skill is needed in the manufacture and testing of drugs, and the costs of formulating current drugs are very high, often hundreds of millions of pounds. Nevertheless, nearly all drugs in circulation have undesirable side effects, and may harm the user. Despite very strict regulation by government agencies, in the USA over 100,000 deaths per annum are attributed to adverse reactions to approved drugs.

Thus according to a fifth aspect of the present invention there is provided a preparation comprising a medically efficacious substance coated or otherwise enclosed by an agent that forms a liquid impermeable but gas permeable layer for use as a medicament.

The inventor has termed this "ActivSignal" (TM) class of drugs.

The agent may be formulated such that the substance cannot cross epithelial barriers in mammals. The agent may be formulated such that the substance cannot cross membrane barriers in mammals. The agent may be formulated such that the substance cannot cross into cells of mammals.

The medically efficacious substance may be selected from the group consisting of: sodium chloride, capsaicin, metformin, salicylic acid, and a derivative of salicylic acid. It may be selected from the group consisting of: a substance endogenous to the body, a food substance, and a drug.

The preparation may be formulated in a variety of ways, including for oral, buccal, nasal, sublingual, rectal, parenteral, topical, dermal or inhalational administration and use, the formulations including nanoparticle and microparticle forms.

For example, the preparation may be in the form of a pill, a tablet, a lozenge, a bolus, a capsule, a caplet, a granule, a nanoparticle, or a microparticle. It may be in granular, nanoparticle or microparticle form as a suspension, a cream, or a paste.

The fifth aspect of the present invention provides for drugs to have a therapeutic effect without the undesirable side effects of drugs put into circulation, such as accumulation, toxicity or possible patient overdose. The invention gives the drug a consistent therapeutic effect as the "dose" is a constant and there is no metabolic change or metabolic by product. The invention may improve the shelf life of drugs by excluding moisture. In the present invention some endogenous body substances may be used for a therapeutic effect, as such substances are formulated not to pass into circulation where they would have no effect.

A preparation according to the present invention may be prepared for use with a patch for holding said preparation near to or against the skin of a patient.

The preparation may be for implantation into the body of a patient.

The agent may be a ceramic, a polymer, a natural wax, or beeswax hardened with cornstarch and talc.

The substance may be sodium chloride, capsaicin, metformin, salicylic acid, or a derivative of salicylic acid.

The substance may be a substance endogenous to the body, a food substance, a plant material, or a drug.

Such preparations may be combined with a preparation of at least one ingredient designed for delivery into solution, particularly with a preparation of at least one ingredient designed for delivery into solution for a therapeutic purpose.

In one embodiment the active ingredient is formulated into oral administration tablets together with the excipients natural beeswax, cornstarch and talc. This method of manufacture is well known to those skilled in the art. Wax matrices are widely employed for drug delivery throughout the pharmaceutical industry because of the low production cost and ease of manufacture. In conventional drug delivery tablet manufacture the proportion of excipients by weight in the tablet is in the range of approximately 1% to 5%, and designed to facilitate immediate or delayed release of the active ingredient into solution. In this most preferred embodiment the excipients, mostly wax plus a small amount of cornstarch and talc, are a proportion of the tablet mixture between 20% and 45% by weight. This novel method of manufacture is designed not to release any of the active ingredient into liquid solution, but the wax matrix is inherently gas permeable.

In a more preferred embodiment, the active ingredient of the tablet is enclosed by a polymer and formulated to be liquid impermeable, but gas permeable. In preferred embodiment, the active ingredient may be enclosed by a ceramic and formulated to be liquid impermeable, but gas permeable. In a fourth embodiment the active ingredient may be enclosed within a metal tablet or capsule, such as perforated stainless steel, allowing passage of a gas but not liquid. Other embodiments include a pill, lozenge, bolus, capsule, caplet, granule or any suitable type, size or shape of manufacture, administered by any route, wherein the active ingredient is protected from contact with liquid but which is potentially contactable by a gas.

It is desirable to test the integrity of the liquid impermeability characteristic of a formulation, and that its physical integrity is preserved during use. A suitable *in vitro* test for a tablet, for example, is agitation in water adjusted to a pH of 3 with hydrochloric acid for three hours, followed by agitation in water adjusted to a pH of 7 with sodium bicarbonate for a further twenty four hours. The medication should not be changed to any significant degree by this test.

*In vivo,* after oral administration, a tablet may tested by being recovered from faeces, and should be found to be unchanged, or only changed to an insignificant degree, after passage through the body.

Drugs formulated according to the present invention may be formulated in nanoparticle or microparticle size. US 4622244 discloses the microencapsulation of an active by a suitable polymer to produce microcapsules of less than 300 microns in size i.e. suitable for injection in a suspension medium by means of small needles customarily employed in medical practice and thereby achieving controlled or sustained release of the active into body circulation. This method of manufacture is well known to those skilled in the art, and in the instance of the present invention the manufacture is achieved by encapsulation by a wax or polymer or other suitable barrier which is liquid impermeable but gas permeable.

Any embodiment may be made up for monotherapy or combined for multiple therapy as convenient. Any embodiment may include more than one active ingredient as convenient. Any embodiment may be combined with a preparation of any other active ingredient prepared for immediate or delayed or selective delivery into solution, as convenient.

Since the therapeutic effect of the invention depends on the presence of the drug in the environment of the body, arrangements can be made to prevent desensitisation over time or the wearing off of the therapeutic effect. In the case of oral administration, desensitisation is avoided by the constant movement of the medicament through the gastrointestinal tract. In the case of administration by other routes it may be necessary for the present invented medicament to be provided and withdrawn at intervals, for example provided for three minutes in each thirty minutes, to avoid desensitisation. For example, a tablet of an ActivSignal class drug may be placed on the skin and withdrawn at intervals by use of a device including an electrical or other energy driven actuator held near to the skin of a subject. The same method is used where it is desired to surgically or otherwise implant an ActivSignal class drug in the body of a subject. Alternatively, very small quantities of the preparations may be used at longer intervals. For example a dermal patch may be provided with two pellets of the drug (the medically efficacious substance) formulated according to the present invention and of 2-3 mm diameter and placed about 30 mm apart with the patch being moved to a different location on the skin after e.g. every twelve hours.

Also provided according to a sixth aspect of the present invention is a method of manufacture of a medicament comprising coating or otherwise enclosing a medically efficacious substance in an agent that forms a liquid impermeable but gas permeable layer.

The various features of the other aspects of the present invention as discussed above apply equally to the sixth aspect of the invention.

It will be appreciated that the amount of the drug used according to the present invention and the physiochemical properties of any agent employed to coat or otherwise enclose the drug will be influenced by the route of administration as well as a number of other factors including the health status of the subject being treated.

The invention will be further apparent from the following description, with reference to the several figures of the accompanying drawings, which show, by way of example only, forms of the present invention. Of the Figures:
- Figure 1: illustrates a patch according to the third aspect of the invention.
- Figure 2: illustrates a device according to the fourth aspect of the invention; and
- Figure 3: illustrates a section view of a device according to the fourth aspect of the invention.

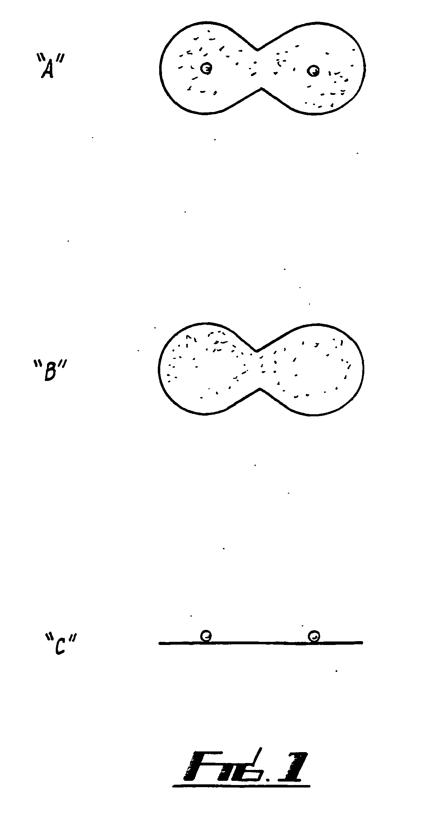

In Figure 1: "A" illustrates a patch according to the third aspect of the invention showing the obverse adhesive coated side comprised of hypoallergenic water resistant plaster with two 2.5 mm diameter spherical granules of coated sodium chloride approximately 30 mm apart and fixed to the adhesive of the plaster; "B" illustrates the reverse side of the same hypoallergenic water resistant plaster; and "C" illustrates a side view of the hypoallergenic water resistant plaster showing two 2.5 mm spherical granules of coated sodium chloride approximately 30 mm apart and fixed to the adhesive of the plaster.

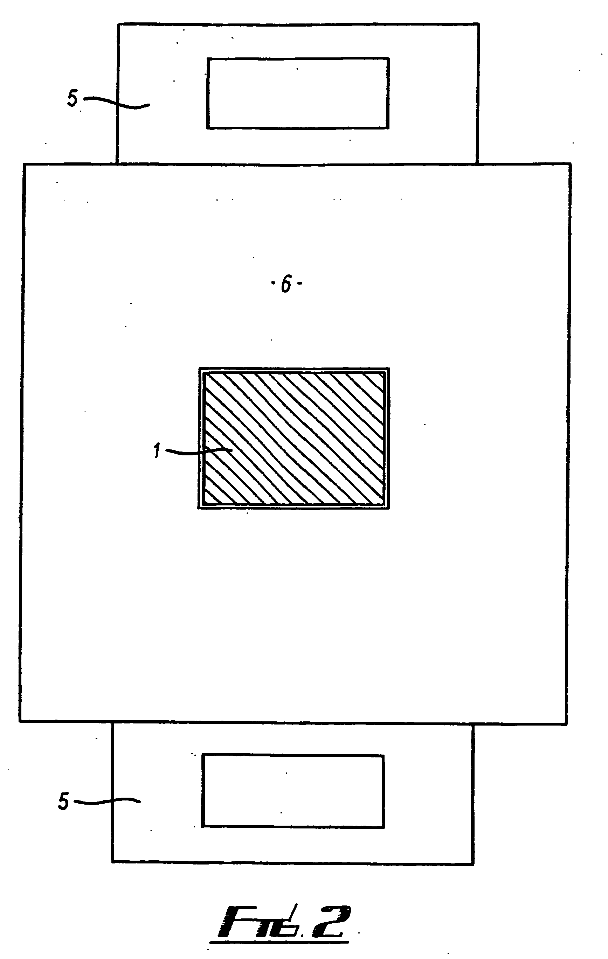

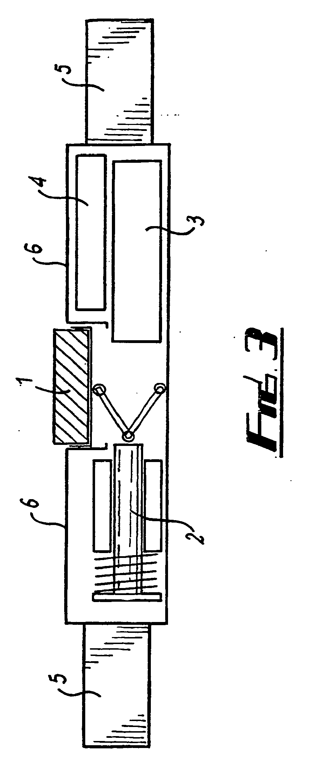

Figures 2 and 3 illustrate a device according to the fourth aspect of the invention. The scale is approximately 3 to 1. These figures illustrate a block of coated sodium chloride approx 12 mm x 13 mm x 4 mm (1); a spring return push rod solenoid (2); a low voltage battery (3); a electronic timer (4); handles to take belt or strapping (5); and a plastic casing for the device to be placed against the skin (6).

### EXAMPLES

### EXAMPLE ONE

### BACKGROUND

Cayenne pepper, a common food ingredient, is extracted from the chilli pepper (Capsicum annum) seed pod. The active ingredient of the pepper is the alkaloid capsaicin. Creams or lotions containing 0.025-0.075 % capsaicin are on sale and have a long history of use in dermatology for the treatment of itching and pain. When applied to the skin, capsaicin causes a burning sensation associated with depletion of neuropeptides from nociceptor nerve endings. Successful suppression of itch by topical administration has been reported for a number of pruritic dermatoses (Reimann S et al., "Topical administration of capsaicin in dermatology for treatment of itching and pain", Hautarzt. 2000 Mar;51(3):164-72; PMID: 10789077).

Atopic eczema (dermatitis) is a highly pruritic skin disease with patches of inflammation, weeping, blistering and bleeding if scratched. Many sufferers have disturbed sleep due to the constant itching. The open nature of the inflammation means that topical capsaicin with its burning sensation cannot be used to relieve eczema pruritus. Indeed topical capsaicin can induce dermatitis.

### METHOD

Cayenne pepper was coated with a formulation of beeswax hardened with a small quantity of cornstarch and talc and compressed to form spherical pills of about 7 mm diameter. According to the present invention these were made to be gas permeable but liquid impermeable ActivSignal class drugs. Samples of the pills were agitated *in vitro* in acidic water for 4 hours, and alkaline water for 24 hours. The pills remained intact and no cayenne pepper was found in the water.

Six adults with atopic eczema (dermatitis) who had complained of pruritus and who were solely using topical preparations for relief were recruited. After informed consent was obtained each was asked to take one of the cayenne ActivSignal class pills at 3 pm and then not use their topical medications until after 8 am the following morning. Each was asked to continue the treatment in the same manner for seven days. Each was asked each following day by telephone to rate their relief from overnight pruritus. Four subjects reported 100% relief on each of the seven nights as a result of the ActivSignal class drug treatment. One subject reported 90% relief and one reported 50% relief as a result of the ActivSignal class drug treatment. No adverse side effects were reported. Two subjects reported a feeling of warmth and slight sweating 2-3 hours after the first occasion of taking the pill.

It is concluded that a gas permeable but liquid impermeable preparation of cayenne pepper according to the present invention is effective for the treatment of eczema pruritus, with no adverse side effects reported.

### EXAMPLE TWO

### BACKGROUND

Metformin, a biguanide, has been available in the USA for the treatment of type 2 diabetes mellitus for nearly 8 years and in Europe for over 20 years. Over this period of time, it has become the most widely prescribed oral anti-hyperglycaemic agent. Its mechanism of action involves the suppression of endogenous glucose production, primarily by the liver. Whether the drug actually has an insulin sensitising effect in peripheral tissues, such as muscle and fat, remains somewhat controversial. Nonetheless, because insulin levels decline with metformin use, it has been termed an 'insulin sensitiser'. Metformin has also been shown to have several beneficial effects on cardiovascular risk factors and it is the only oral anti-hyperglycaemic agent thus far associated with decreased macrovascular outcomes in patients with diabetes. Cardiovascular disease, impaired glucose tolerance and the polycystic ovary syndrome are now recognised as complications of the insulin resistance syndrome, and there is growing interest in the use of metformin for these extraordinarily common metabolic disorders. While diet and exercise remain the cornerstone of therapy for insulin resistance, pharmacological intervention by use of metformin is now a well used alternative.

Metformin, however, is thought to sometimes accumulate in the body and thus increase the risk of lactic acidosis, a potentially fatal condition. Metformin therefore is considered to be contraindicated in many chronic hypoxemic conditions that may be associated with lactic acidosis, such as cardiovascular, renal, hepatic and pulmonary disease, and advancing age.

### METHOD

Metformin 250 mg was coated with a formulation of beeswax hardened with a small quantity of cornstarch and talc and compressed to form spherical pills of about 7 mm diameter. According to the present invention these were made to be gas permeable but liquid impermeable ActivSignal class drugs. Samples of the pills were agitated *in vitro* in acidic water for 4 hours, and alkaline water for 24 hours. The sample pills remained intact. On dissection no liquid was found to have entered the sample pills.

Five adults suffering from diabetes type 2 controlled by metformin alone were recruited. The group had been diagnosed with fasting plasma glucose (FPG) in the-range 10 to 15 mmol/l before commencing the metformin treatment. Current dosages ranged from 500 mg metformin twice daily to 850 mg metformin three times daily. The five adults were controlling their FPG to below 8 mmol/l and were taking weekly measurements with results in the range 5 to 8 mmol/l. Three of the five recalled having a metallic taste in the mouth occasionally and all five reported occasional abdominal discomfort as side-effects of taking metformin.

With informed consent, the five adults agreed to substitute the ActivSignal metformin for the regular metformin at the rate of one ActivSignal metformin pill for each regular metformin tablet they were currently taking, for a period of four weeks.

During the four week trial all of the five adults reported that they were controlling their FPG to below 8 mmol/l whilst taking the ActivSignal metformin. No side effects were reported.

It is concluded that ActivSignal metformin according to the present invention has the equivalent therapeutic effect to regular metformin for persons suffering from moderate diabetes type 2, but that ActivSignal metformin has reduced or no side effects. In addition, since ActivSignal metformin is not released into the body there can be no accumulation, so that ActivSignal metformin is likely to be able to be used where currently contraindicated for the therapy of persons with chronic conditions that may be associated with lactic acidosis.

### EXAMPLE THREE

### BACKGROUND

Aspirin is the acetyl derivative of salicylic acid that is used to lower fever, relieve pain, reduce inflammation, and thin the blood. Common conditions treated with aspirin include headache, muscle and joint pain, and the inflammation caused by rheumatic fever and arthritis. Aspirin is believed to act against fever, pain, and inflammation by interfering with the synthesis of specific prostaglandins in the body. Because of its ability to inhibit the formation of blood clots, aspirin is also used in low doses to prevent heart attack and stroke and to control unstable angina. The drug's usefulness in preventing certain cancers, the dangerous high blood pressure that sometimes occurs during pregnancy (toxemia), and migraine headaches is also under investigation.

Normal dosage may cause nausea, vomiting, diarrhoea, or gastrointestinal bleeding. Large doses cause acid-base imbalance and respiratory disturbances and can be fatal, especially in children. Aspirin also has been linked to the development of Reyes' syndrome (a combination of acute encephalopathy and fatty infiltration of internal organs) in children who have taken it for viral infections. Acetaminophen (paracetamol) which does not cause gastric irritation, lowers fever and relieves pain but does not reduce inflammation, is often substituted for aspirin. Ibuprofen may be used instead of aspirin for up to ten days without consultation with a physician. Ibuprofen may have similar side effects to aspirin although this is less common.

Salicylic acid or 2-hydroxybenzoic acid, C₆H₄(OH)CO₂H, is colourless, crystalline organic carboxylic acid used as an oral analgesic up to the end of the nineteenth century, until the invention of the less irritating acetyl derivative, aspirin. Other derivatives of salicylic acid are used as an active ingredient of many topical preparations including sun creams, toothpaste, antiseptics and food. Aspirin is the most widely used medication in the world with over 80 billion doses sold annually in the USA alone, and aspirin is an active ingredient in over fifty over-the-counter medications.

### METHOD

Pharmaceutical grade aspirin was hydrolysed to salicylic acid and then coated with beeswax hardened with cornstarch and talc and compressed to form pills of about 6 mm diameter. According to the present invention these were made to be gas permeable but liquid impermeable ActivSignal class drugs. Samples of the pills were agitated *in vitro* in acidic water for 4 hours, and alkaline water for 24 hours. The sample pills remained intact. On dissection no liquid was found to have entered the sample pills.

Twelve adults were recruited who were taking aspirin or acetaminophen (paracetamol) or ibuprofen ad lib for the relief of mild to moderate arthritic pain, at up to the maximum recommended dose per day, namely 12 x aspirin 300 mg, or 8 x acetaminophen 500 mg or 6 x 200 mg ibuprofen. Some of the group were taking combined aspirin and acetaminophen up to the combined recommended daily dosage.

With informed consent members of the group agreed to substitute their regular analgesic with ActivSignal salicylic acid pills for three weeks. They were advised to start, when required, with one ActivSignal salicylic acid pill per day or two (one morning, one evening) if required. They were advised, if necessary, they could take (one at a time) up to six pills per day with a minimum two hour interval. At the end of the trial six persons reported that the ActivSignal salicylic acid pills were more effective at relieving pain than their regular analgesic. A further five persons reported that the ActivSignal salicylic acid pills gave about the same level of pain relief as their regular analgesic. These eleven persons had taken either one, two or three ActivSignal salicylic acid pills on most days. None had found the need to take more than three of the pills on any day. All reported that the pain relief seemed to be longer lasting with the ActivSignal salicylic acid pills than with their regular analgesic. No side effects were reported. One person found the ActivSignal salicylic acid pills less effective than the ibuprofen she was normally taking, and dropped out of the trial.

It is concluded that ActivSignal salicylic acid according to the present invention is an effective and long lasting analgesic with no reported side effects.

### EXAMPLE FOUR

### BACKGROUND

Essential hypertension is one of the major health problems of the developed world, affecting over 20% of the adult population. Essential hypertension is defined as persistent high pressure of unknown cause. Untreated essential hypertension can lead to heart attack (myocardial infarction), congestive heart failure, other heart damage, arteriosclerosis, kidney damage, stroke and loss of vision.

The inventor has found that the main cause of essential hypertension is the prevalence of asymptomatic miliaria profunda in the population of advanced societies, this disease causing inflammation which raises pressure in skin capillaries. The miliaria is a result of over conservation of sodium by sweat gland ducts. It follows that by using ActiveSignal class drugs containing sodium the over conservation can be reversed, and the miliaria and thus the essential hypertension is prevented.

The classification of blood pressure in adults by the World Health Organisation and the International Society of Hypertension (revised 1999) is as listed in Table 1.

**TABLE 1:**

| Classification | Systolic | | Diastolic |
|---|---|---|---|
| Optimal | <120 | and | <80 |
| Normal | <130 | and | <85 |
| High-normal | 130-139 | or | 85-89 |
| Mild hypertension | 140-159 | or | 90-99 |
| Moderate hypertension | 160-179 | or | 100-109 |
| Severe hypertension | >180 | or | >110 |

All values are mmHg. Measurements are taken with subjects in the sitting position.

Sodium chloride or common salt occurs naturally in many parts of the world. By mass, sodium chloride is 60.663% elemental chlorine and 39.337% sodium. Sodium chloride crystals are cubic in form and are readily available as a pure chemical.

Sodium chloride is an endogenous substance of the mammalian body, and essential to the maintenance of life, but ordinary intake of the chemical has never been found to have a therapeutic effect. Indeed restriction of sodium chloride is recommended for persons with essential hypertension.

### METHOD

Pure sodium chloride was coated with beeswax hardened with cornstarch and talc and compressed to form pills of about 6mm diameter ActivSignal class drug and granules of about 2 mm diameter ActivSignal class drug. Two each of the granules were fixed to an adhesive hypoallergenic patch at a distance of about 30 mm.

Following informed consent nine persons with measured mild, moderate or severe hypertension were asked to participate in a trial. Before commencement of the trial, the blood pressure of each person was measured after the subject had been at rest seated for fifteen minutes. After a further ten minutes the blood pressure was measured again and the average of the two systolic readings and the average of the two diastolic readings was noted. Measurements were taken using the Omron 705IT Blood Pressure monitor, a clinically validated machine. Small, medium and large cuffs were available and selected according to the manufacturers instructions.

The subjects were then asked to fix the patch with the two ActivSignal class drug granules anywhere on the front of their abdomen. Further, a new patch was fixed in a different position after each twenty four hours for a further three days and the old patch discarded.

The subjects were also given one of the pill ActivSignal class drugs taken orally with about 200 ml water on the first, and third days of the trial.

Blood pressures were taken in the same manner after 1, 2, 4 and 6 days of the trial.

At the start of the trial four of the persons had mild hypertension, three had moderate hypertension and two had severe hypertension. After four days, measurements of blood pressure as listed in Table 2 show that following treatment the systolic blood pressure had been reduced by 27% and the diastolic pressure by 18%.

After four days six subjects were now within the "optimum" classification of blood pressure, and two were now within the "normal" classification. One of the subjects originally having severe hypertension was now classified as having mild hypertension. After six days, as shown in Table 2, the benefits of the treatment persisted after the treatment had been discontinued.

These data illustrate that the use of sodium chloride as an ActivSignal class drug according to the present invention may easily and rapidly reduce blood pressure to within normal limits so that the subjects can no longer be considered hypertensive. The treatment has the effect of restoring skin blood capillaries to their natural free flowing function.

The invention has the effect of resetting blood pressure to what is considered normal. Essential hypertension is known to be only a slowly progressive disease and it can therefore be anticipated that following treatment the subjects are unlikely to become hypertensive again for many months or some years, when the treatment can be repeated.

Compared with treatments with current pharmaceutical products which may need to be taken for a lifetime, which have unpleasant side effects, and which do not treat the underlying disease, the present invention is a much swifter, more effective and less costly treatment of essential hypertension with no known side effects.

Results are shown in Table 2.

**Table 2**

| Subj No. M/F Age | | Before Trial | After 1 day | After 2 days | After 4 days | Percent reduction | After 6 days |
|---|---|---|---|---|---|---|---|
| (01)F55 | Syst | 158 | 139 | 122 | 108 | 31% | 106 |
| | Diast | 88 | 74 | 67 | 62 | 30% | 58 |
| (02) M62 | Syst | 162 | 135 | 121 | 103 | 35% | 102 |
| | Diast | 89 | 78 | 70 | 58 | 33% | 47 |
| (03) M55 | Syst | 157 | 122 | 122 | 118 | 24% | 120 |
| | Diast | 77 | 78 | 78 | 68 | 9% | 72 |
| (04) F49 | Syst | 196 | 160 | 124 | 122 | 37% | 128 |
| | Diast | 96 | 93 | 81 | 78 | 18% | 78 |
| (05) F50 | Syst | 157 | 122 | 122 | 123 | 21% | 122 |
| | Diast | 72 | 72 | 72 | 70 | 2% | 70 |
| (06) M65 | Syst | 175 | 167 | 157 | 154 | 12% | 155 |
| | Diast | 90 | 90 | 90 | 85 | 5% | 86 |
| (07) M52 | Syst | 188 | 149 | 131 | 128 | 31% | 125 |
| | Diast | 108 | 96 | 88 | 78 | 27% | 79 |
| (08) F54 | Syst | 146 | 135 | 129 | 114 | 15% | 119 |
| | Diast | 88 | 83 | 83 | 77 | 12% | 80 |
| (09) M52 | Syst | 162 | 140 | 133 | 116 | 22% | 116 |
| | Diast | 98 | 90 | 89 | 80 | 18% | 78 |

## Claims

1. A composition for use as a medicament, comprising a at least one medically efficacious substance coated with an agent wherein the agent forms a liquid impermeable but gas permeable layer surrounding said substance and preventing the passage of said substance through said coating, wherein in-use said substance is not released into solution in the body.

2. The composition according to claim 1 wherein said agent is selected from the group consisting of: a ceramic, a polymer, a natural wax, and beeswax hardened with cornstarch and talc.

3. The composition according to claim 1 or 2, in a form selected from the group consisting of: a pill, a tablet, a lozenge, a bolus, a capsule, a caplet, a granule, a nanoparticle, and a microparticle.

4. The composition according to claim 3, being in granular, nanoparticle or microparticle form selected from the group consisting of: a suspension, a cream, and a paste.

5. The composition according to any of the preceding claims, prepared for use with a patch for holding said composition near to or against the skin of a patient.

6. The composition according to any of claims 1-4, prepared for implantation Into the body of a patient

7. The composition according to any of the preceding claims, wherein said substance is selected from the group consisting of: a substance endogenous to the body, a food substance, a plant material, and a drug.

8. The composition according to any of the preceding claims, wherein said substance is selected from the group consisting of: sodium chloride, capsaicin, metformin, salicylic acid, and a derivative of salicylic acid.

9. A composition according to claim 8, wherein said substance comprises sodium chloride.

10. The composition according to claim 9 wherein said sodium chloride is in crystal form.

11. The composition according to claim 10 wherein said agent encapsulates sodium chloride to form a sphere.

12. The composition according to claim 11 wherein said sphere is of a diameter between 1 mm and 10 mm.

13. The composition according to claim 11 or 12 wherein said sphere comprises sodium chloride crystals coated with beeswax hardened with cornstarch and talc.

14. The composition according to any of the preceding claims, combined with a preparation of at least one ingredient designed for delivery into solution.

15. The composition according to any of claims 1-13 combined with a preparation of at least one ingredient designed for delivery into solution for a therapeutic purpose.

16. A patch suitable for adherence to skin containing a composition according to any of the preceding claims.

17. The patch according to claim 16 comprising a sticking plaster suitable for adherence to skin.

18. The patch according to claim 17 comprising a hypoallergenic water resistant plaster.

19. The patch according to claim 17 or 18 in the form of a figure eight.

20. The patch according to claim 19 further comprising two spherical granules of sodium chloride.

21. A device comprising a holder adapted for holding a composition according to any of claims 1-15, an energy source and an actuator driven by the energy source for temporarily and at intervals placing said composition on or near to the skin of a subject.

22. The device according to claim 21 adapted to be worn around the abdomen or thorax of a subject.

23. The device according to either of claims 21 or 22 wherein the energy source is a low voltage battery.

24. The device according to any of claims 21-23 further comprising an electronic timer.

25. The device according to any of claims 21-24 wherein the actuator is a spring return push-rod solenoid.

26. A preparation for treatment of a body comprising a composition according to any of claims 1-15.

27. The use of a composition according to any of claims 1-15 in a method of manufacture of a medicament.

28. A method of manufacture of a medicament comprising coating, or otherwise enclosing, a at least one medically efficacious substance within an agent that forms a liquid impermeable, but gas permeable layer which surrounds the substance and prevents the passage of the substance through said layer, wherein in-use said substance is not released into solution in the body.

## Patentansprüche

1. Zusammensetzung zur Verwendung als Medikament, wobei die Zusammensetzung mindestens eine medizinisch wirksame Substanz aufweist, die mit einem Mittel beschichtet ist; wobei das Mittel eine flüssigkeitsundurchlässige, aber gasdurchlässige Schicht bildet, welche die Substanz umgibt und den Durchgang der Substanz durch die Beschichtung verhindert, wobei die Substanz im Gebrauch nicht in Lösung im Körper freigesetzt wird.

2. Zusammensetzung nach Anspruch 1, wobei das Mittel aus der Gruppe ausgewählt ist, die aus einer Keramik, einem Polymer, einem Naturwachs und mit Maisstärke und Talkum gehärtetem Bienenwachs besteht.

3. Zusammensetzung nach Anspruch 1 oder 2 in einer Form, die aus der Gruppe ausgewählt ist, die aus einer Pille, einer Tablette, einer Pastille, einem Bolus, einer Kapsel, einem Dragee, einem Granulat, einem Nanoteilchen und einem Mikroteilchen besteht.

4. Zusammensetzung nach Anspruch 3, die in Form von Granulat, Nanoteilchen oder Mikroteilchen vorliegt und aus der Gruppe ausgewählt ist, die aus einer Suspension, einer Creme und einer Paste besteht.

5. Zusammensetzung nach einem der vorstehenden Ansprüche, die zur Verwendung mit einem Pflaster präpariert wird, um die Zusammensetzung nahe oder an der Haut eines Patienten zu halten.

6. Zusammensetzung nach einem der Ansprüche 1-4, die zur Implantation in den Körper eines Patienten präpariert wird.

7. Zusammensetzung nach einem der vorstehenden Ansprüche, wobei die Substanz aus der Gruppe ausgewählt ist, die aus einer körpereigenen Substanz, einer Nahrungssubstanz, einem Pflanzenmaterial und einem Medikament besteht.

8. Zusammensetzung nach einem der vorstehenden Ansprüche, wobei die Substanz aus der Gruppe ausgewählt ist, die aus Natriumchlorid, Capsalcin, Metformin, Salicylsäure und einem Salicylsäurederivat besteht.

9. Zusammensetzung nach Anspruch 8, wobei die Substanz Natriumchlorid aufweist.

10. Zusammensetzung nach Anspruch 9, wobei das Natriumchlorid in Kristallform vorliegt.

11. Zusammensetzung nach Anspruch 10, wobei das Mittel Natriumchlorid einkapselt, um eine Kugel zu bilden.

12. Zusammensetzung nach Anspruch 11, wobei die Kugel einen Durchmesser zwischen 1 mm und 10 µm hat.

13. Zusammensetzung nach Anspruch 11 oder 12, wobei die Kugel Natriumchloridkristalle aufweist, die mit Bienenwachs beschichtet sind, das mit Maisstärke und Talkum gehärtet ist.

14. Zusammensetzung nach einem der vorstehenden Ansprüche in Kombination mit einem Präparat aus mindestens einem Bestandteil, das für die Abgabe in Lösung konstruiert ist.

15. Zusammensetzung nach einem der Ansprüche 1-13 in Kombination mit einem Präparat aus mindestens einem Bestandteil, das für die Abgabe in Lösung zu einem therapeutischen Zweck konstruiert ist.

16. Zum Anhaften an die Haut geeignetes Pflaster, das eine Zusammensetzung nach einem der vorstehenden Ansprüche enthält.

17. Pflaster nach Anspruch 16, das ein zum Anhaften an die Haut geeignetes Heftpflaster aufweist.

18. Pflaster nach Anspruch 17, das ein hypoallergenes wasserfestes Pflaster aufweist.

19. Pflaster nach Anspruch 17 oder 18 in Form einer Ziffer acht.

20. Pflaster nach Anspruch 19, das ferner zwei kugelförmige n Natriumchloridkörnchen aufweist.

21. Vorrichtung, die einen Behälter aufweist, der zur Aufnahme einer Zusammensetzung nach einem der Ansprüche 1-15, einer Energiequelle und eines durch die Energiequelle angetriebenen Betätigungselements eingerichtet ist, um die Zusammensetzung zeitweilig und in Intervallen an oder nahe der Haut eines Patienten anzubringen.

22. Vorrichtung nach Anspruch 21, die so eingerichtet ist, dass sie um den Abdomen oder Thorax eines Patienten getragen wird.

23. Vorrichtung nach Anspruch 21 oder 22, wobei die Energiequelle eine Niederspannungsbatterie ist.

24. Vorrichtung nach einem der Ansprüche 21-23, die ferner einen elektronischen Zeitschalter aufweist.

25. Vorrichtung nach einem der Ansprüche 21-24, wobei das Betätigungselement eine Magnetspule mit Stößelstange und Federrückführung ist.

26. Präparat zur Behandlung eines Körpers, das eine Zusammensetzung nach einem der Ansprüche 1-15 aufweist.

27. Verwendung einer Zusammensetzung nach einem der Ansprüche 1-15 bei einem Verfahren zur Herstellung eines Medikaments.

28. Verfahren zur Herstellung eines Medikaments mit Beschichtung oder anderem Einschluss mindestens einer medizinisch wirksamen Substanz in einem Mittel, das eine flüssigkeitsundurchlässige, aber gasdurchlässige Schicht bildet, welche die Substanz umgibt und den Durchgang der Substanz durch die Schicht verhindert, wobei die Substanz im Gebrauch nicht in Lösung im Körper freigesetzt wird.

## Revendications

1. Composition pour une utilisation en tant que médicament, comprenant au moins une substance médicalement efficace enrobée avec un agent, dans laquelle l'agent forme une couche imperméable aux liquides mais perméable aux gaz entourant ladite substance et prévenant le passage de ladite substance à travers ledit enrobage, dans laquelle à l'usage ladite substance n'est pas libérée en solution dans le corps.

2. Composition selon la revendication 1, dans laquelle ledit agent est choisi dans le groupe constitué de: une céramique, un polymère, une cire naturelle et une cire d'abeilles durcie avec de l'amidon de maïs et du talc.

3. Composition selon la revendication 1 ou 2, sous une forme choisie dans le groupe constitué de: une pilule, un cachet, une pastille, un bolus, une capsule, un caplet, un granule, une nanoparticule et une microparticule.

4. Composition selon la revendication 3, qui est sous une forme granulaire, nanoparticulaire ou microparticulaire choisie dans le groupe constitué de: une suspension, une crème et une pâte.

5. Composition selon l'une quelconque des revendications précédentes, préparée pour une utilisation avec un patch pour maintenir ladite composition près de ou contre la peau d'un patient.

6. Composition selon l'une quelconque des revendications 1-4, préparée pour une implantation dans le corps d'un patient.

7. Composition selon l'une quelconque des revendications précédentes, dans laquelle ladite substance est choisie dans le groupe constitué de: une substance endogène pour le corps, une substance alimentaire, une matière végétale et un médicament.

8. Composition selon l'une quelconque des revendications précédentes, dans laquelle ladite substance est choisie dans le groupe constitué de: chlorure de sodium, capsalcine, metformine, acide salicylique et un dérivé d'acide salicylique.

9. Composition selon la revendication 8, dans laquelle ladite substance comprend du chlorure de sodium.

10. Composition selon la revendication 9, dans laquelle ledit chlorure de sodium est sous forme de cristaux.

11. Composition selon la revendication 10, dans laquelle ledit agent encapsule le chlorure de sodium pour former une sphère.

12. Composition selon la revendication 11, dans laquelle ladite sphère est d'un diamètre entre 1 mm et 10 mm.

13. Composition selon la revendication 11 ou 12, dans laquelle ladite sphère comprend des cristaux de chlorure de sodium enrobés avec une cire d'abeilles durcie avec de l'amidon de maïs et du talc.

14. Composition selon l'une quelconque des revendications précédentes, combinée avec une préparation d'au moins un ingrédient conçu pour une distribution en solution.

15. Composition selon l'une quelconque des revendications 1-13, combinée avec une préparation d'au moins un ingrédient conçu pour une distribution en solution pour un objectif thérapeutique.

16. Patch approprié pour une adhérence à la peau contenant une composition selon l'une quelconque des revendications précédentes.

17. Patch selon la revendication 16 comprenant un plâtre adhésif approprié pour une adhérence à la peau.

18. Patch selon la revendication 17 comprenant un plâtre résistant à l'eau hypoallergénique.

19. Patch selon la revendication 17 ou 18 sous la forme d'un tour de huit.

20. Patch selon la revendication 19 comprenant en outre deux granules sphériques de chlorure de sodium.

21. Dispositif comprenant un récipient adapté pour contenir une composition selon l'une quelconque des revendications 1-15, une source d'énergie et une commande actionnée par la source d'énergie pour placer temporairement et par intervalles ladite composition sur ou près de la peau d'un sujet.

22. Dispositif selon la revendication 21 adapté pour être porté autour de l'abdomen ou du thorax d'un sujet.

23. Dispositif selon l'une ou l'autre des revendications 21 et 22, dans lequel la source d'énergie est une batterie à basse tension.

24. Dispositif selon l'une quelconque des revendications 21-23 comprenant en outre un chronomètre électronique.

25. Dispositif selon l'une quelconque des revendications 21-24, dans lequel la commande est un solénoïde à poussoir à rappel par ressort.

26. Préparation pour le traitement d'un corps comprenant une composition selon l'une quelconque des revendications 1-15.

27. Utilisation d'une composition selon l'une quelconque des revendications 1-15 dans un procédé de fabrication d'un médicament.

28. Procédé de fabrication d'un médicament comprenant l'enrobage, ou une autre façon d'enfermer, d'au moins une substance médicalement efficace avec un agent qui forme une couche imperméable aux liquides mais perméable aux gaz qui entoure la substance et prévient le passage de la substance à travers ladite couche, dans lequel à l'usage ladite substance n'est pas libérée en solution dans le corps.
